# EUROPÄISCHE PATENTANMELDUNG

(11) **EP 1 319 366 A1**
(43) Veröffentlichungstag der Anmeldung: **18.06.2003**
(21) Anmeldenummer: 01129329.7
(22) Anmeldetag: 14.12.2001
(51) Int. Cl.: A61B 5/06

(54) **Magnetische Katheternavigation**

(71) Anmelder: BrainLAB AG, 85551 Kirchheim/Heimstetten (DE)
(72) Erfinder: Vilsmeier, Stefan, 6330 Kufstein (AT); Witte, Jens, 80997 München (DE); Birkenbach, Rainer, 85586 Poing (DE)
(74) Vertreter: Schwabe - Sandmair - Marx

(57) **Zusammenfassung**

Die Erfindung betrifft eine Vorrichtung zum Verbringen eines Katheters in das Körperinnere mit einem in der Katheterröhre (1) einsetzbaren Stützstilett (5), welches die Form der Katheterröhre (1) beim Eindringen in das Körperinnere aufrecht erhält, bei der am Stützstilett (5) eine positionsgebende Magnettrackingeinrichtung (7, 8, 9, 10) angeordnet ist, die mittels einer magnettreckingbasierten Navigationseinrichtung (11, 12) erfassbar ist.

## Beschreibung

Die vorliegende Erfindung betrifft im Allgemeinen die magnetische Katheternavigation und insbesondere eine Vorrichtung zum Verbringen eines Katheters in das Körperinnere, mit einem in die Katheterröhre einsetzbaren Stützstilett, welches die Form der Katheterröhre beim Eindringen in das Körperinnere aufrechterhält.

Solche Katheter bzw. Vorrichtungen werden beispielsweise dann verwendet, wenn Flüssigkeit aus dem Körperinneren abgeleitet werden soll. Wenn sich z.B. bei einem Patienten nach einer Kopfverletzung im Inneren des Gehirns ein Ödem, also ein Bereich bildet, in dem sich Flüssigkeit anreichert, muss diese möglichst schnell abgeleitet werden, um größere Hirnschädigungen durch zu großen intercerebralem Druck zu vermeiden. Dazu werden derzeit in der Unfallmedizin Katheter verwendet, die durch eine zu erzeugende Knochenöffnung per Hand so in das Gehirn des Patienten eingesetzt werden, dass die Spitze des Katheters in der Zone zu liegen kommt, wo sich die Flüssigkeitsansammlung bildet. Der Katheterschlauch ist an der Spitze mit Perforierungen versehen, durch die die Flüssigkeit in das Schlauchinnere gelangen kann, um dann abtransportiert zu werden. Ein weiterer Anwendungsbereich ist der Fall des angeborenen Hydrocephalus, bei dem eine Überproduktion von Hirnwasser zu einem überhöhten Hirninnendruck führt, so dass dieses Wasser permanent abgeleitet werden muss. Im Verlauf wird der Katheter dabei vom Gehirn unter der Haut bis in die Bauchregion gelegt.

Erfahrenes Personal verwendet derzeit zum Einbringen der Katheterspitze in die richtige Position noch eine Methode, bei der der richtige Eindringwinkel und die richtige Eindringstelle beispielsweise anhand der Knochennähte der Kopfknochen manuell identifiziert werden. Diese Methode ist jedoch möglicherweise selbst bei erfahrenem Ärztepersonal ungenau und unerfahrene Ärzte haben noch größere Schwierigkeiten, die exakte Positionierung der Katheterspitze vorzunehmen. Gerade im Hirnbereich ist aber jede Abweichung kritisch, da die Folgen von Fehleinsetzungen, nämlich Schädigungen des gesunden Gewebes und mangelnder Flüssigkeitsabtransport fatal sein können.

Es ist deshalb die Aufgabe der vorliegenden Erfindung, eine Vorrichtung zum Verbringen eines Katheters in das Körperinnere bereitzustellen, die eine genaue Platzierung der Katheterspitze und eine zuverlässig richtige Einbringung durch das Ärztepersonal sicherstellt. Insbesondere soll eine solche Behandlung in einem Umfeld möglich sein, in dem nur geringer apparativer Aufwand zur Verfügung steht.

Diese Aufgabe wird erfindungsgemäß durch den Gegenstand des Patentanspruchs 1 gelöst. Die Unteransprüche definieren bevorzugte Ausführungsformen der vorliegenden Erfindung.

Gemäß der Erfindung wird am Stützstilett für die Katheterröhre eine positionsgebende Magnettrackingeinrichtung angeordnet, die mittels einer magnettrackingbasierten Navigationseinrichtung erfassbar ist. Mit anderen Worten wird das ohnehin notwendige Stabilisationsmittel zur Einbringung der Katheterröhre gleichzeitig als Positionierungshilfe verwendet, die in einem Umfeld verwendet werden kann, in dem eine magnettrackingbasierte Navigationseinrichtung zur Verfügung steht. Solche magnettrackingbasierten Navigationseinrichtungen benötigen kein aufwändiges Kamerasystem mehr wie zum Beispiel optisch basierte Navigationseinrichtungen und eignen sich damit in idealer Weise für die Kathetemavigation im erfindungsgemäßen Sinne. Computertomographie- oder Kernspintomographiedaten werden bei der Registrierung und der aktuellen Erfassung der Körperteilkoordinaten verwendet. Dazu wird von dem Patienten eine CT- oder MR-Aufnahme gemacht, bevor er behandelt wird.

Am Bildschirm des Magnettracking-Navigationssystems kann der Arzt, der den Katheter einführt, nunmehr kontrollieren, ob der Zielpunkt getroffen wurde oder ob noch Korrekturen vorzunehmen sind. Außerdem kann das Navigationssystem schon Vorschläge zur Eindringlänge, zum Einführungspunkt oder zum Einführungswinkel machen bzw. eine falsche Einbringung durch Warnhinweise anzeigen. Dabei stehen dem Personal zwei- und dreidimensionale Darstellungen auf dem Bildschirm des Navigationssystems zur Verfügung.

Der Katheter wird mit dem zur Stabilisierung notwendigen, in die Katheterröhre eingeführten Stilett in das Körperinnere eingebracht, und gerade dieses Stilett kann erfindungsgemäß die Magnettrackingeinrichtung tragen. Wenn der Katheter einmal positioniert ist, kann das Stilett aus dem hinteren Ende herausgezogen werden, wo dann über einen Adapter ein Abführschlauch an die Katheterröhre anbringbar ist.

Die angesprochene Magnettrackingeinrichtung kann Miniaturspulen aufweisen, wobei vorteilhafterweise mindestens zwei Spulen vorgesehen sind, die unterschiedliche Raumausrichtungen aufweisen. Mit einer solchen Spulenanordnung lässt sich eine in allen Freiheitsgraden bestimmbare Positionsverfolgung des Katheters realisieren. Es soll hier auch angemerkt sein, dass es bei der vorliegenden Erfindung grundsätzlich wichtig ist, den Eindringwinkel sowie die Eindringtiefe festzustellen oder zu navigieren, so dass prinzipiell die Bereitstellung einer einzigen Miniaturspule an dem Stilett genügen kann, da die Navigation beispielsweise im Bezug auf die Rotation des Stiletts bzw. der Katheterröhre um die Längsachse relativ unkritisch ist.

Bei einer Ausführungsform der vorliegenden Erfindung ist eine erste Spule am spitzenabgewandten Bereich des Stiletts angeordnet, während eine zweite Spule an der Stilettspitze angeordnet ist. Gerade die Anordnung einer Spule an der Stilettspitze ermöglicht eine sehr genaue Erfassung der Eindringtiefe und der Eindringrichtung, wenn die andere Spule relativ weit davon entfernt am spitzen abgewandten Ende des Stiletts positioniert ist.

Gemäß einer anderen Ausführungsform der Erfindung ist ein Spulenpaar mit Spulen unterschiedlicher Raumausrichtung am spitzenabgewandten Ende des Stiletts angeordnet, insbesondere in oder an einer Griffeinrichtung des Stiletts, wo sich ausreichend Platz für die Anordnung auch größerer Spulen bietet.

Natürlich kann jedwede Kombination der Spulenanzahl und Spulenpositionierung gewählt werden, solange die Navigationsaufgaben erfüllt werden können.

Bei einer Ausführungsform der Erfindung ist das Stilett zur Signal- oder Energieübertragung eines Kabels mit der Navigationseinrichtung verbunden. So können beispielsweise Erregerströme an die Spulen weitergegeben werden oder induzierte Ströme aus den einzelnen Spulen abgelesen werden.

Gemäß einer weiteren Ausführungsform weist das Stilett eine Einrichtung zum drahtlosen Signaltausch mit der Navigationseinrichtung auf, sowie insbesondere eine eigene Energieversorgung. Hierdurch lässt sich ein "kabelloses" Stilett realisieren, das dem Behandlungspersonal einen sehr weitgehenden Freiraum bei der Behandlung und eine große Bewegungsfreiheit einräumt.

Vorteilhafterweise ist das Stillet zusammen mit der Magnettrackingeinrichtung als Einweg- bzw. Wegwerfware ausgebildet und wird insbesondere zusammen mit der Katheterröhre oder separat davon steril verpackt bereitgestellt.

In besonders bevorzugter Ausführungsform ist der Katheter ein Kopf- bzw. Hirnkatheter.

Um die Bewegung des Körperteils, in das der Katheter eingesetzt werden soll, bei der Navigation mit berücksichtigen zu können, wird in fester positioneller Zuordnung zu diesem Körperteil ein Referenzsensor angebracht. Dieser Referenzsensor ist vorteilhafterweise wiederum eine Spulenanordnung, die räumlich getrackt werden kann und ihre Bewegungsdaten über Kabel oder Funk an das Navigationssystem übermittelt.

Es gibt- je nach betreffendem Patientenkörperteil - einige Möglichkeiten, einen solchen Referenzsensor zu befestigen. Es könnte beispielsweise ein Headset verwendet werden, wie es aus der Navigation für Hals-, Nasen-, Ohrenbehandlungen bekannt ist, ein elastisches Band (Stirnband) oder ein Klebeband mit Referenzsensor können verwendet werden. Eine bevorzugte Ausführungsform der vorliegenden Erfindung benutzt einen angeklebten Referenzsensor, insbesondere wenn es sich bei dem zu setzenden Katheter um einen Kopf- bzw. Hirnkatheter handelt. In einem solchen Fall ist es von Vorteil, den Referenzsensor an einem Zahn am Oberkiefer zu befestigen. Dabei kann der Referenzsensor einerseits direkt auf den Zahn des Patienten geklebt werden, beispielsweise mit einem Zahnkleber, wie er auch zur Befestigung von Zahnspangen verwendet wird. In besonders bevorzugter Ausführungsform wird der Referenzsensor über einen Zwischenadapter an einem Oberkieferzahn befestigt, d. h. lediglich der Adapter wird fest angeklebt und der Referenzsensor kann dann auf den Adapter angebracht, beispielsweise dort angesteckt werden.

Ein Patient mit einem solchen Referenzsensor auf einem Zahn kann ohne weiteres bei der magnetischen Navigation den Mund schließen. Allgemeine Vorteile sind die einfache Handhabung, die sehr hohe Genauigkeit und die Tatsache, dass ein Verrutschen des Sensors während der Behandlung nicht möglich ist. Die Ausführungsform mit am Zahn angeklebten Zwischenadapter hat noch dazu den Vorteil, dass der Sensor erst kurz vor der Behandlung angebracht werden muss und mehrfach wiederverwendet werden kann.

Die Erfindung wird nun im weiteren anhand einer Ausführungsform näher erläutert. Hierzu wird auf die anliegenden Zeichnungen Bezug genommen; es zeigen:
- Figur 1: eine schematische Darstellung einer erfmdungsgemäßen Vorrichtung mit Katheter und eingeführtem, magnettracking-navigierbarem Stilett; und
- Figur 2: eine schematische Darstellung für das technische Umfeld im Rahmen einer erfindungsgemäßen, magnetischen Katheternavigation.

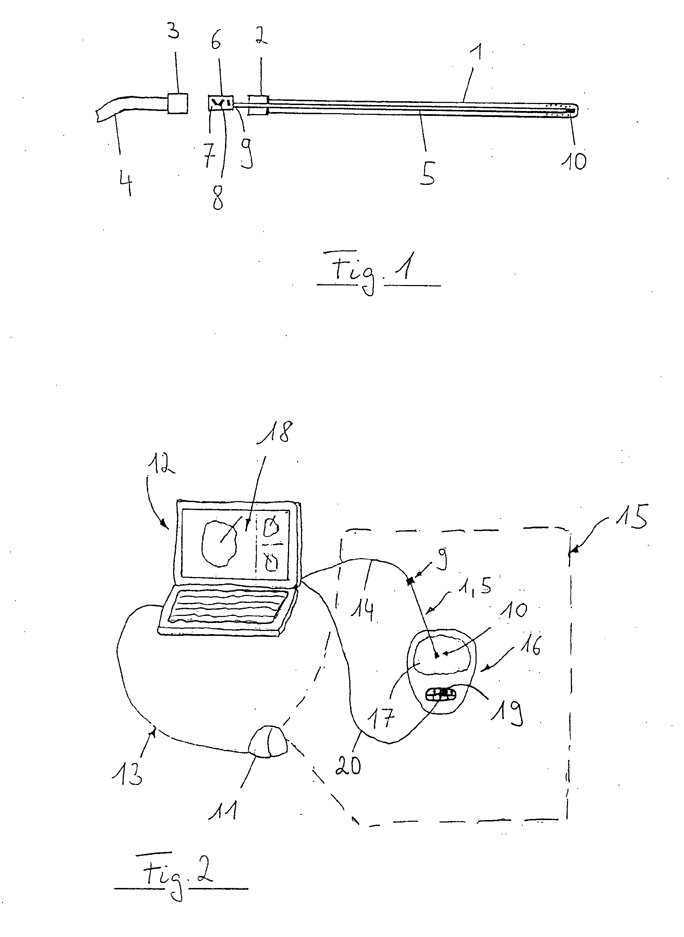

In der Figur 1 ist die erfindungsgemäße Vorrichtung schematisch dargestellt. Sie umfasst eine Katheterröhre 1, die an ihrem links dargestellten Ende einen Anschlussadapter und an ihrem rechts dargestellten Ende, der Spitze, nicht bezeichnete Perforierungen aufweist, durch welche Flüssigkeit in das Innere der Katheterröhre gelangen kann. Die Katheterröhre ist sehr dünn ausgebildet und besteht aus einem weichen Kunststoffmaterial. Links in Figur 1 ist noch ein Katheterschlauch 4 gezeigt, der über seinen Adapter 3 mit dem Anschlussadapter 2 der Katheterröhre 1 verbunden werden kann, wenn das Stilett 5 nach dem Einsetzen des Katheters wieder herausgezogen wird.

Das gerade genannte Stilett 5 ist in die Katheterröhre 1 eingesetzt, um diese beim Einbringen der Katheterröhre in das Körperinnere zu stützen. Es reicht im wesentlichen bis an die Spitze der Katheterröhre 1 und kann an seinem spitzenabgewandten Ende eine Art Griffstück 6 aufweisen, das an einem Teil des Stiletts 5 befestigt ist, welcher hinten über dem Anschlussadapter 2 hervorsteht. Das Stilett 5 ist aus einem sehr dünnen, aber stabilen Material hergestellt.

In der Figur 1 sind ferner mögliche Anordnungen für Miniaturspulen dargestellt, die zur magnetischen Navigation am Stilett angebracht werden. Einerseits kann sich im Griffstück ein Spulenpaar 7, 8 befinden, wobei die Spule 7 und die Spule 8 jeweils in unterschiedlichen Raumrichtungen angeordnet sind, im vorliegenden Beispiel stehen sie V-förmig zueinander. Ebenfalls als Option dargestellt ist das Spulenpaar 9, 10, wobei die Spule 10 in Axialrichtung an der Spitze (rechts) des Stiletts 5 angeordnet ist, während die Spule 9 senkrecht zur Axialrichtung im Griffstück 6 steht. Mit Hilfe solcher Spulenanordnungen lässt sich in bekannter Weise eine dreidimensionale Navigation sowie ein dreidimensionales Tracking des Stiletts 5 und damit auch der umgebenden Katheterröhre 1 durchführen, bis die Spitze der Katheterröhre am richtigen Punkt im Körperinneren sitzt. Danach wird das Stilett am Griffstück 6 aus der Katheterröhre 1 herausgezogen und die Katheterröhre 1 und der Schlauch 4 können mit Hilfe der Adapter 2 und 3 verbunden werden; die überschüssige Flüssigkeit kann dann abfließen.

In der Figur 2 ist das technische Umfeld für eine Methode mit der erfindungsgemäßen Vorrichtung schematisch dargestellt. Bereitgestellt wird ein Navigationssystem mit der Computer- und Anzeigeeinheit 12, die einen Schirm 18 und nicht bezeichnete Eingabeelemente sowie Steckanschlüsse für weitere Komponenten aufweist. An einem solchen Steckanschluss ist mittels eines Kabels 13 ein Feldgenerator 11 verbunden, der ein Magnettracking-Feld aufbaut, dessen Umrisse gestrichelt angedeutet und mit dem Bezugszeichen 15 versehen sind. Innerhalb dieses Feldes 15 befindet sich der schematisch dargestellte Kopf 16 eines Patienten, in dessen ebenfalls schematisch dargestellter Gehirnstruktur sich eine Flüssigkeitsansammlung gebildet hat.

Ferner umfasst das System noch einen sogenannten Referenzsensor 19, der die Bewegung des Patientenkörperteils mit ausführt und deshalb an diesem Körperteil fest angeordnet ist. Im vorliegenden Fall ist der Referenzsensor 19, der bewegungsfest mit dem Kopf 16 des Patienten verbunden sein soll, an einem Oberkieferzahn des Patienten angebracht. Er besteht ebenfalls aus einer Spulenanordnung, die eine räumliche Positionserfassung im magnetischen Trackingsystem gestattet. Der Sensor 19 ist ebenfalls mittels eines Kabels 20 mit der Computerund Anzeigeeinheit verbunden, so dass die Bewegungen des Kopfes 16, die während des Kathetersetzens gemacht werden, im Navigationssystem bekannt sind und in die Navigation einberechnet werden können. Es ist bei der magnetischen Navigation kein Problem, wenn der Patient seinen Mund über dem Referenzsensor 19 schließt. Im vorliegenden Fall ist der Referenzsensor 19 über einen am Zahn angeklebten Adapter angebracht, so dass er vor der Behandlung nur dort aufgesetzt werden muss und mehrfach wiederverwendbar ist. Es ist natürlich auch ein direktes Ankleben des Referenzsensors 19 an einen Zahn des Patienten im O-berkieferbereich möglich; in beiden Fällen wird ein Verrutschen des Sensors während der Behandlung ausgeschlossen.

Bei der dargestellten Ausführungsform ist mit einem Kabel 14 ein mit einem Stilett versehener Katheterschlauch in das Hirn eingebracht worden. Die beiden Bauteile sind mit 1, 5 bezeichnet. In übertrieben großer Darstellung ist am Spitzenende und am spitzenabgewandten Ende des Stiletts jeweils eine Spule 10 bzw. 9 aufgezeigt, die so angeordnet sind, wie die entsprechenden Bauteile in Figur 1. In dem dargestellten Zustand kann der Arzt, welcher den Katheter setzen soll, auf der Anzeigevorrichtung 18 kontrollieren, wie weit und in welchem Winkel der Katheter bereits eingesetzt ist und entsprechende Korrekturen vornehmen oder das richtige Einsetzen des Katheters steuern. Die Informationen hierüber erhält das Navigationssystem aus den Positionen der Spulen 9 und 10 im durch den Generator 11 erzeugen Feld 15. Nach dem richtigen Einsetzen wird dann, wie oben schon beschrieben, das Stilett 5 entfernt und die Flüssigkeitsabführung kann beginnen.

Durch die vorliegende Erfindung hat deshalb auch unerfahrenes Behandlungspersonal die Möglichkeit, einen solchen Katheter genau und ohne unnötige Verletzung gesunden Gewebes zu setzen, während erfahrene Ärzte immer kontrollieren können, ob das Setzen des Katheters erfolgreich war.

## Patentansprüche

1. Vorrichtung zum Verbringen eines Katheters in das Körperinnere, mit einem in die Katheterröhre (1) einsetzbaren Stützstilett (5), welches die Form der Katheterröhre (1) beim Eindringen in das Körperinnere aufrecht erhält, **dadurch gekennzeichnet, dass** am Stützstilett (5) eine positionsgebende Magnettrackingeinrichtung (7, 8, 9, 10) angeordnet ist, die mittels einer magnettrackingbasierten Navigationseinrichtung (11, 12) erfassbar ist.

2. Vorrichtung nach Anspruch 1, **dadurch gekennzeichnet, dass** die Magnettrackingeinrichtung Miniaturspulen (7, 8, 9, 10) aufweist.

3. Vorrichtung nach Anspruch 2, **dadurch gekennzeichnet, dass** mindestens zwei Spulen vorgesehen sind, die unterschiedliche Raumausrichtungen aufweisen.

4. Vorrichtung nach Anspruch 3, **dadurch gekennzeichnet, dass** eine erste Spule (9) am spitzenabgewandten Bereich des Stiletts (5) angeordnet ist, während eine zweite Spule (10) an der Stilettspitze angeordnet ist.

5. Vorrichtung nach Anspruch 3, **dadurch gekennzeichnet, dass** ein Spulenpaar mit Spulen (7,8) unterschiedlicher Raumausrichtung am spitzenabgewandten Ende des Stiletts (5) angeordnet ist, insbesondere in oder an einer Griffeinrichtung (6) des Stiletts (5).

6. Vorrichtung nach einem der Ansprüche 1 bis 5, **dadurch gekennzeichnet, dass** das Stilett (5) zur Signal- und oder Energieübertragung mittels eines Kabels (14) mit der Navigationseinrichtung (11, 12) verbunden ist.

7. Vorrichtung nach einem der Ansprüche 1 bis 5, **dadurch gekennzeichnet, dass** das Stilett (5) eine Einrichtung zum drahtlosen Signaltausch mit der Navigationseinrichtung (11, 12) aufweist, sowie insbesondere eine eigene Energieversorgung.

8. Vorrichtung nach einem der Ansprüche 1 bis 7, **dadurch gekennzeichnet, dass** das Stilett (5) zusammen mit der Magnettrackingeinrichtung als Einweg- bzw. Wegwerfware ausgebildet ist und insbesondere zusammen mit der Katheterröhre (1) oder separat davon steril verpackt bereitgestellt wird.

9. Vorrichtung nach einem der Ansprüche 1 bis 8, **dadurch gekennzeichnet, dass** der Katheter ein Kopf- bzw. Hirnkatheter ist.

10. Vorrichtung nach einem der Ansprüche 1 bis 9, **dadurch gekennzeichnet, dass** die magnettrackingbasierte Navigationseinrichtung einen Referenzsensor (19) umfasst, der fest positioniert an dem Patientenkörperteil angebracht wird, in das der Katheter eingebracht wird.

11. Vorrichtung nach Anspruch 10, **dadurch gekennzeichnet, dass** der Referenzsensor (19) dann, wenn der Katheter ein Kopf- bzw. Hirnkatheter ist, am Oberkiefer befestigt wird, insbesondere an einem Zahn des Oberkiefers.

12. Vorrichtung nach einem der Ansprüche 10 oder 11, **dadurch gekennzeichnet, dass** der Referenzsensor (19) mittels eines fest am Oberkiefer bzw: Zahn angebrachten Adapters abnehmbar befestigt wird.
